# EUROPEAN PATENT APPLICATION

(11) **EP 2 583 681 A1**
(43) Date of publication of application: **24.04.2013**
(21) Application number: 10852809.2
(22) Date of filing: 08.06.2010
(51) Int. Cl.: A61K 31/7056, A61P 31/14

(54) **ORAL VETERINARY COMPOSITION FOR SALMONIDS, COMPRISING 1-BETA-D-RIBOFURANOSYL-1H-1,2,4-TRIAZOLE-3-CARBOXAMIDE AND USE THEREOF FOR THE TREATMENT OF INFECTIOUS SALMON ANAEMIA (ISA) IN SALMONIDS**

(71) Applicant: Laboratorio De Diagnostico Gam, S.a., Región Metropolitana, Santiago 7500652 (CL)
(72) Inventor: SANDINO, Ana, María, Santiago 7500652 (CL); MLYNARZ ZYLBERBERG, Geraldine, Santiago 7500652 (CL); JASHES MORGUES, Matilde, Santiago 7500652 (CL); SPENCER OSSA, Eugenio, Santiago 7500652 (CL)
(74) Representative: Carvajal y Urquijo, Isabel
(86) International application number: PCT/IB2010/052548
(87) International publication number: WO 2011/154772

(57) **Abstract**

ABSTRACT

The invention relates to a novel oral veterinary composition for salmonids, comprising 1-beta-D-ribofuranosyl-1 H1,2,4-triazole-3-carboxamide and to the use thereof for the treatment of infectious salmon anaemia (ISA) caused by the infectious' salmon anaemia or ISA virus in salmonids.

## Description

### DESCRIPTION OF THE INVENTION

The present invention relates to a novel oral veterinary composition for salmonids, comprising ribavirin and to the use thereof for the treatment of infectious salmon anemia caused by the infectious salmon anemia or ISA virus in salmonids.

### BACKGROUND OF THE INVENTION

Infectious Salmon Anemia (ISA) is a viral exclusive in culture disease of the Atlantic salmon (Salmo salar L.), discovered in 1984 in Norway *(*Thorud and Djupvik, Infectious anemia in Atlantic salmon (Salmo salar L.), Bull. Eur. Assoc. Fish Pathol. 8: 109-111, 1988) and it is characterized by provoking high mortality in fish. The most common signs are severe anemia; exophthalmos; ascites; petechial hemorrhage in viscera, adipose tissue and skin; and hemorrhagic necrosis in the liver of infected fish *(*Falk and Dannevig, infectious salmon anemia Demonstration of (ISA) viral antigens in cell cultures and tissue sections. Vet. Res 26: 499-504, 1995*;* Falk et al, Characterization of infectious salmon anemia virus, an orthomyxo-like virus isolated from Atlantic salmon (Salmo salar L.), J. Virol. 71: 9016 -9023, 1997, Munir and Kibenge, Detection of infectious salmon anemia virus by real-time RT-PCR. J. Virol. Methods 117: 37-47, 2004*).* In the economic field, this disease has caused great losses in salmon producing countries where it has been declared, such as Norway, Canada, Scotland, Faroe Islands and the United States (Kibenge et al, Isolation and identification of infectious salmon anemia virus (ISAV) from Coho salmon in Chile, Dis Aquat Organ 45, 9-18, 2001*;* Lovely et al, First identification of infectious salmon anemia virus in North America with haemorrhagic kidney syndrome, Dis Aquat Organ 35, 145-148, 1999; Falk and al, Identification and characterization of viral structural proteins of infectious salmon anemia virus, J Virol 78, 3063-3071, 2004*).* Moreover, in the case of Norway, the high mortality produced losses covering almost the entire production, reporting up to 80% reductions in the total production of the country. Chile, a country that until recently was free of the disease, has also been attacked by the virus, partly due to the globalization of markets and on the other hand due to the intense production conditions at the farms, which allows an easy dissemination, representing a problem often difficult to control. Finally the pathogen agent was first detected in Chile in 2001 *(*Kibenge et al, Isolation and identification of infectious salmon anemia virus (ISAV) from Coho salmon in Chile, Dis Aquat Organ 45, 9-18, 2001*).*

The etiologic agent of this disease is the Infectious Salmon Anemia Virus (ISAV). It belongs to the *Orthomyxoviridae* family, it has 8 segments of single-stranded genomic RNA of negative polarity, coding for 8 structural proteins and 2 nonstructural proteins. The genomic organization of ISAV has situated it in a new genus, the Isavirus or Aquaorthomyxovirus *(*Krossoy et al, The putative polymerase sequence of infectious salmon anemia virus Suggests a new genus Within the Orthomyxoviridae, J Virol 73, 2136-2142, 1999). The 8 genomic RNA segments are attached to multiple copies of the viral nucleoprotein (NP), a copy of the RNA polymerase complex formed by proteins PB1, PB2 and PA is situated at the ends 3'; which altogether are called ribonucleoproteins. A membrane envelope in which the glycoproteins Hemagglutininesterase (HE) and fusion (F) are inserted surrounds the protean capsid, which is formed by matrix protein M1 and M2, *(*Aspehaug et al, Characterization of the infectious salmon anemia virus fusion protein, J Virol 79, 12544-12553, 2005*).*

The ISAV replicative cycle is very similar to the influenza A virus, where the HE protein identifies a cellular receptor containing 4-O-acetyl-sialic acid (Hellebo et al, Infectious salmon anemia virus binds to and hydrolyzes especificamente 4-O- acetylated sialic acids, J Virol 78, 3055-3062, 2004*).* Subsequently the particle is entered into the cell in vessels covered with clathrin, which are fused to endosomes, providing the necessary acidic environment for the fusion among membranes, both viral and endosomal, allowing the stripping of the virus. The viral ribonucleoproteins are forwarded to the nucleus of the cell, where viral transcription starts. The viral mRNA produced at the nucleus is translated in the cellular cytoplasm, returning to the nucleus only the NP, PB1, PB2 and PA proteins; allowing the beginning of viral replication and subsequently, the formation of ribonucleoproteins. The assembly of mature viral particles is performed in the cell membrane toward which the glycoproteins are headed, HE and F, and also M proteins, which will be the NP receptors, finally releasing the virions by a budding process,

The study of diseases caused by microorganisms allows the design of different control strategies, either with early diagnosis, confronting the spread of the disease or mainly attacking its origin, that is, the same microorganisms. In the case of virus, it is essential to study both the replicative cycle in general and the function and structure of each of the viral proteins. Through these studies it has been found that the orthomyxovirus undergoes a high rate of mutation and recombination forming new strains from one year to another. This is the main reason why prevention through vaccination does not guarantee protection against infection with these agents, leading to develop alternative therapies to prevent the spread of infection within and between organisms. The design and testing of compounds that interfere with the replication cycle of the virus is a relevant area of study for the control of the orthomyxovirus as ISAV.

To date, approximately 40 antiviral compounds have been approved for its use in humans, primarily for the treatment of infections caused by the human immunodeficiency virus (HIV), hepatitis B virus (HBV) and herpes virus. On the other hand, the number of approved antiviral compounds that can be used for the treatment of infections caused by RNA is limited. Among these are, without considering treatment for HIV, the M2 channel inhibitors, amantadine and rimantadine, and the neuraminidase inhibitors, oseltamivir and zanamivir for influenza; and ribavirin for the treatment of respiratory syncytial virus (RSV), the hepatitis C virus (HCV) and is also being used for the treatment of Lassa fever *(Revised In:* Leyseen et a/, Molecular Strategies to inhibit the replication of RNA viruses, Antivir. Res 78: 9-15, 2008*).*

Ribavirin is a synthetic nucleoside whose chemical name is 1-beta-D-ribofuranosyl-1H-1 ,2,4-triazole-3-carboxamide having the following structural formula (Formula I):

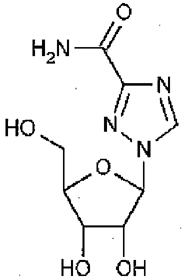

Ribavirin is a broad-spectrum inhibitor of RNA virus replication, which has been approved for treatment of HCV infections in combination with interferon and for the treatment of RSV infections in pediatric aerosol form. It has also been used experimentally for other conditions, including Lassa fever (Khan et al, New Opportunities for field research, on the pathogenesis and treatment of Lassa fever, Antivir. Res 78: 103-115, 2008*),* CCHF virus *(*Ergonul, Treatment of Crimean-Congo hemorrhagic fever. Antivir res 78: 125-131, 2008) and Hantavirus *(*Jonsson et al. hantavirus pulmonary syndrome Treatment of, Antivir. res 78: 162-169, 2008)*.* While it has been found that the compound inhibits *in vitro* replication of some viruses studied, most of them being RNA virus, it has been shown to have a protective effect only in some animal models. Also, the power of the *in vitro* activity of ribavirin may vary considerably depending on the nature of the virus *(*Graci and Cameron, Mechanism of action of ribavirin against distinct viruses, Rev. Med Virol. 16, 37-48, 2006*).*

It has been reported that most of RNA viruses are sensitive to the activity of ribavirin *in vitro,* but only some of them are more susceptible than others. In general, ribavirin is not very potent as an antiviral medication regularly showing EC50 values (effective concentration 50%) of 1 µM or even higher. Specifically, *in vitro* antiviral activity has been proved against bunyaviruses including CCHF virus (hemorrhagic fever Crimean-Congo), Rift Valley fever virus, and Hantavirus. It also inhibits the replication of coronaviruses, including coronavirus-SARS and flaviviruses, but has shown not to be effective in animals experimentally infected with these viruses *(*Watts y cols, Inhibition of Crimean-Congo hemorrhagic fever viral infectivity yields in vitro by ribavirin. Am. J. Trop. Med. Hyg. 41: 581-585, 1989*;* Huggins, Prospects for treatment of viral hemorrhagic fevers with ribavirin, a broad-spectrum antiviral drug. Rev. Infect. Dis. 11 (Suppl. 4) S750-S761, 1989*;* Severson y cols, Ribavirin causes error catastrophe during Hantan virus replication. J. Virol. 77: 481-488, 2003*;* Saijo y cols, Inhibitory effect of mizoribine and ribavirin on the replication of severe acute respiratory syndrome (SARS)-associated coronavirus: Antivir. Res. 66: 159-163, 2005*;* Barnard y cols, Enhancement of the infectivity of SARS-CoV in BALB/c mice by IMP dehydrogenase inhibitors, including ribavirin. Antivir. Res. 71: 53-63, 2006*;* Neyfs y cols, Use the yellow fever virus vaccine strain 17D for the study of strategies for the treatment of Bellow fever virus infections. Antivir. Res. 30 (2-3): 125-132, 1996*).* At a similar way, ribavirin is not effective in animal models infected with flovirus *(*Huggins, Prospects for treatment of viral hemorrhagic fevers with ribavirin, a broad-spectrum antiviral drug. Rev Infect. Dis. 11 (Suppl. 4) S750-S761, 1989*).* It It has also been observed that ribavirin is effective *in vitro* and *in vivo* against RSV, a paramyxovirus, and it reveals relative sensitivity in cell cultures of other paramyxoviruses, the Nipah virus, but only limited efficacy in animals models has been observed (Snell, ribavirin therapy for Nipah virus infection. J. Virol. 78: 10211, 2004*,* Georges-Courbot et al, Poly-(I)-poly (C12U) but not ribavirin prevents it death in a hamster model f Nipah virus infection, Antimicrob. Agents Chemother. 50:1768-1772, 2006*).*

It has also been evaluated the effect of ribavirin on VHSV virus (Viral Hemorrhagic Septicemia Virus), in EPC cell cultures *(Ephitelioma papulosum cyprini).* Cells were infected with the virus and treated with 1 to 25 µg/ml Ribavirin. The results revealed a strong inhibition of the virus at concentrations of 5, 10 and 25 µg/ml. They also show a high inhibition of viral RNA accumulation when 25 µg/ml are added at 0 hours post infection, occurring RNA inhibition of 99.8% at 10 hours post infection. This report concludes that the measuring method employed (RT-PCR in real time) can be used in combination with the classical methods to study the progression of the infection and the kinetics of virus replication, but there is however, not always a correlation of *in vitro* results with the protection given to the fish, so it is necessary to conduct tests *in vivo (*Moroccan et al,, Assessment of the inhibitory effect of ribavirin on the rainbow trout rhabdovirus VHSV by real-time reverse-transcription PCR , Vet. Microbiol. 122: 52-60, 2007*).* In this case, a test was conducted with a virus that infects the rainbow trout and its effect *in vitro* was assayed. There are no reports *in vivo* and not anything suggests that the same effects on cells in culture could be seen in fish.

On the other hand the antiviral effect of ribavirin in cell cultures infected with IPNV virus (Infectious Pancreatic Necrosis Virus) has been evaluated (Jashés et al. Inhibitors of infectious pancreatic necrosis virus (IPNV) replication, Antiviral Res 29: 309-312 , 1996*,* Hudson et al, The Efficacy of amantadine and other antiviral compounds against salmonid two viruses in vitro, Antiviral Res 9: 379-385, 1988). It was observed that this antiviral is capable of inhibiting viral replication *in vitro* of the IPN virus with an EC50 of 0.5 g/mL and a CC₅₀ of 100 µg/mL, but it was not the most effective antiviral and the *in vivo* activity was no longer evaluated (Jashés et al. Inhibitors of infectious pancreatic necrosis virus (IPNV) replication, Antiviral Res 29: 309-312, 1996*).*

In an *in vivo* study reported in 1980, the effect of ribavirin on IPNV infected rainbow trout was analyzed. The compound was supplied through a solution' in the tank where the fish were held by exposure to the antiviral once during two hours. The administration was performed in increasing concentrations to the fish into two separate batches of 6 tanks each. The results revealed a decreased rate of dead fish in about 5%, but there was not a linear effect over the concentration of the antiviral used. Also the higher dose of ribavirin administered, 400 µg, produced no greater decrease in the rate of death. It is concluded that higher doses of ribavirin do not produce a greater decrease in the quantity of dead fish. An alternative, treatment option would be a sustained exposure. to antiviral in order to decrease significantly the death rate of infected fish. However it is suggested that the costs involved would be very expensive and most fish farm owners would be hostile to initiate any antiviral treatment before the existence of an apparent viral illness *(*Savan and Dobos. Effect of Virazole on rainbow trout fry Salmo gairdneri Richardsowinfected with infectious pancreatic necrosis virus, J. Fish Dis., 3: 437-440, 1980). No further testing is reported, so that the usefulness of ribavirin in the treatment of IPNV infected trout has not been demonstrated.

There have been several proposals of molecular mechanisms responsible for the antiviral activity of ribavirin *(*Hong and Cameron, pleiotropic Activities Mechanisms of antiviral ribavirin, Prog Drug Res 59: 47-69, 2002*; Revised:* Leyseen et al, Molecular Strategies to inhibit the replication of RNA viruses, Antivir. Res 78: 9-15, 2008*).* These mechanisms include: (1) depletion of intracellular levels of GTP by inhibition of intracellular IMP dehydrogenase caused by 5'-monophosphate metabolite of ribavirin, (2) inhibition of the viral polymerase activity, caused by the 5'-triphosphate metabolite of ribavirin, (3) inhibition of the viral capsid through inhibition of the guaniltransferase activity caused by the 5'-triphosphate ribavirin, (4) inhibition of viral helicase causing the process known as catastrophic error resulting of the accumulation of mutations, some pf them being lethal, in the viral genome.

In the present, it is still in debate the magnitude of the contribution of the catastrophic error, of the depletion of intracellular levels of GTP and other proposed mechanisms of antiviral activity of ribavirin, and the way they would contribute in the *in vivo* activity of this antiviral.

As it has already been mentioned, ribavirin is used in the chronic treatment of hepatitis C in humans, in association with interferon alpha. The information available for the product Rebetol®, containing ribavirin as active ingredient, establishes that the FDA (Food and Drug Administration) alerts about an important primary toxicity of this compound, because it triggers hemolytic anemia in patients treated with the product and can lead to a worsening of cardiac disease causing both fatal and non-fatal myocardial infarction.

From the available information to date there is not an effective veterinary composition comprising ribavirin, useful in the treatment of salmonids infected with the ISA virus.

To this regard, the need for a veterinary composition including an effective antiviral as ribavirin, useful for the treatment of fish infected with ISA virus is required. It is a problem in the world of aquaculture that does not have an effective and efficient solution nowadays. A solution that could actually stop and reverse the infections caused by this virus, otherwise causing great losses, not only for the producer but also to all consumers whom finally get the product.

The fact of the need of an effective and efficient veterinary composition is also related to the cost of treatment, so as not to increase the price of the final product, in order to give the possibility to dispense a product not only when the disease has spread but also, and as has been suggested for other pathologies treated with antiviral drugs, to be able to dispense it in the early stages of infection, thus allowing more protection by reducing the propagation of the replication stage in which the virus is. This would prevent healthy fish from being infected by cohabitation, blocking the spread and eventually stopping the disease.

From the available data, there is not a veterinary composition capable of inhibiting the ISA virus and even more, there is no composition that could do it *in vivo,* or in naturally infected fish.

As it has been mentioned previously, considering the high mutation presented by the ISA virus that leads to a change in vaccines every year because the effectiveness decrease, the problem should be addressed by looking at the properties of the etiologic agent remaining in time and that are vulnerable to other mechanisms. In view of the ISA virus shares certain characteristics with the influenza virus, the inventors concluded that an efficient way to control the disease could be use of known efficient antivirals to inhibit these viruses. But surprisingly, inventors found that out of all antiviral tested, only ribavirin showed to be really effective *in vitro.*

Ribavirin is a broad-spectrum antiviral compound, but the background and the available data do not suggest that it might be useful for the treatment of this disease, even more considering that it has been reported that one of its most important toxic effects is hemolytic anemia. An expert in the art would never even think about using it for the treatment of an infection caused by ISAV, disease characterized by the development of severe anemia, among other complications, which altogether are ultimately fatal to the fish.

Contradicting all predicted ideas, the inventors of the present invention have surprisingly found that an oral veterinary composition comprising ribavirin is effective in the treatment of fish infected with ISA virus.

### BRIEF DESCRIPTION OF THE INVENTION

The present invention relates to composition of exclusively veterinary use comprising ribavirin, in a pharmaceutical form of oral powder to be incorporated into the normal food pellet for fish through an oil impregnation process, with the dual purpose of facilitating its oral administration and on the other hand, improving bioavailability in the target species.

This veterinary composition comprising ribavirin is useful for the treatment of fish infected with the virus of infectious salmon anemia or ISAV. Ribavirin will be referred to hereinafter as such or as "Antiviral".

In a preferred embodiment, the concentration of ribavirin in the formulation is from 0.5% to 5% by weight related to the total weight of the composition.

In another preferred embodiment, the composition comprising acceptable veterinary excipients for salmonids to be dispensed orally.

In other preferred embodiments, the composition comprising excipients such as spray dried lactose, partially pregelatinized corn starch, lactose monohydrate, corn starch, colloidal silicon dioxide, or mixtures thereof.

In another preferred embodiment, the composition comprising partially pregelatinized, corn starch, as an excipient.

In a specific embodiment, the concentration of ribavirin in the formulation is 5% by weight relative to the total weight of the composition.

### DESCRIPTION OF THE FIGURES

Figure 1: Viral viability in different concentrations of oseltamivir.
Figure 2: Viral viability in different concentrations of shikimic acid.
Figure 3: Viral viability in different concentrations of antiviral.
Figure 4: Cell viability in different concentrations of antiviral.
Figure 5: Cumulative Mortality in fish treated with the antiviral in different oral doses. This figure shows the cumulative mortality observed in the 5 tanks of the test. Fish in tanks C1, C2 and C3 are controls that did not receive antiviral. The fish in tanks T1 and T2 received doses of 800 *µ*g/mL and 400 *µ*g/mL of the compound.
Figure 6: Total average viral load after of the fish treatments in tanks C3, T1 and T2.
Figure 7: Average viral load in live fish samples. These fish were killed by euthanasia at the end of the assay. Viral loads of tanks C3, T1 and T2 are shown.
Figure 8: Average viral load in dead fish samples from the tanks C3, T1 and T2. These fish died during the assay by ISA virus.
Figure 9: Average viral load in infected fish samples from the tanks C3, T1 and T2. These fish were infected by intraperitoneal injection.
Figure 10: Viral load in uninfected fish samples from the tanks C3, T1 and T2. These fish were infected by cohabitation with intraperitoneally pre- infected fish.
11A and 11B: Photograph of necropsy in fish of the tank C1.
12A and 12B: Photograph of necropsy in fish of the tank C2.
Figure 13 A: Photograph of necropsy in fish of the tank C3.
Figure 13 B: Close-Up of the photography of necropsy of the Figure 13 A.
Figure 14 A: Photograph of necropsy of a fish of the tank T1 intraperitoneally infected by ISA virus. This fish was treated with 800 *µ*g/mL Antiviral.
Figure 14 B: Photograph of necropsy of two fish of the tank T1 infected by cohabitation with ISA virus. This fish was treated with 800 *µ*g/mL Antiviral.
Figure 15 A: Photograph of necropsy of a fish of the tank T2 intraperitoneally infected by ISA virus. This fish was treated with 400 *µ*g/mL Antiviral.
Figure 15 B: Photograph of necropsy of a fish of the tank T2 infected by the virus ISA cohabitation. This fish was treated with 400 *µ*g/mL Antiviral.
Figure 16: Average hematocrit. The hematocrit in fish of the tanks C1, C2, C3, T1 and T2 is shown.
Figure 17: Mortality in fish with and without treatment. The cumulative mortality of fish from different tanks (No. 1 to No. 8), under treatment and control is shown.
Figure 18: Hematocrit and hemoglobin values. The average values found in the treated fish and in both healthy and infected controls are shown.

### DETAILED DESCRIPTION OF THE INVENTION

### INHIBITION OF VIRAL REPLICATION ASSAYS

### SHK-1 cell culture

The SHK-1 cells derived from the Atlantic salmon kidney were grown at 15 °C in growth medium (Medium Leibovitz L-15, 4 mM L-glutamine, 40 mM 2-mercaptoethanol, 50 mg/mL gentamicin sulfate, 8% SFB).

### ISA virus spread

Monolayers of SHK-1 cells grown for 3 days up to a confluence of 60% approximately, were inoculated with a 1/10 dilution of a virus isolated in a culture of SHK-1 cells from the heart of a naturally infected fish in southern Chile, in infection medium (Leibovitz L-15 without FBS, 4 mM L-glutamine, 40 mM 2-mercaptoethanol, 50 *µ*g/mL gentamicin). After 4 hours of incubation at 15 °C the inoculum was replaced by growth medium. Infected monolayers were incubated afterwards at 15 °C for 3 days. The presence of ISA virus in the inoculum was checked by RT-PCR in real time.

### Inhibition Assay of the replicative cycle of ISAV

Monolayers of SHK-1 cells were grown in 6-well plates, 9.6 cm². The cells were propagated by no more than 3 days and were infected with ISAV. After 4 hours of incubation with virus, the medium was replaced by growth medium including various dilutions of different compounds to be tested (Antiviral, oseltamivir or shikimic acid). After 3 days of post-infection the cell supernatant was collected and subjected to extraction of viral RNA. Viral replication was qualitatively analyzed with RT-PCR quantification in real time.

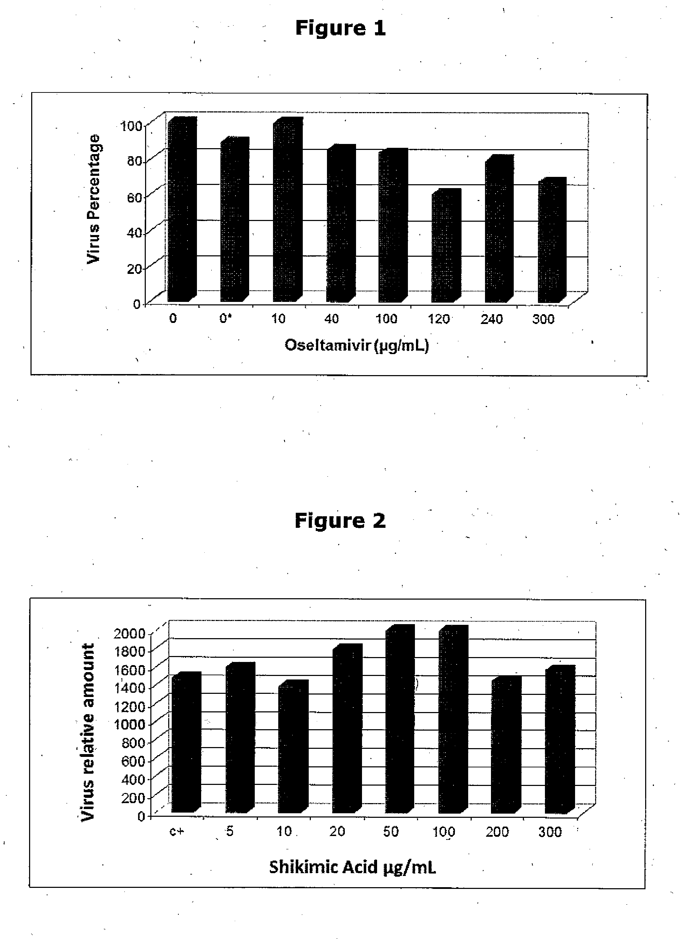

Viral viability was assessed in cells infected with ISA virus, in different antiviral concentrations proved to be effective in the influenza treatment. It was observed that both oseltamivir, a well-known antiviral used in the treatment of influenza, as its precursor, shikimic acid, were little effective in inhibiting viral replication. In the case of oseltamivir no more than 60% of inhibition was obtained for the replication of ISA virus in concentrations as high as 300 *µ*g/mL (Figure 1). On the other hand for influenza viruses A and B, EC₅₀ ranging from 0.03 ng/mL to 2.84 ng/mL have been reported *(*Mai Le et al, Isolation of drug-resistant H5N1 virus, Nature 437: 1108, 2005 Calligari et al, Inhibition of viral Group-1 and Group-2 neuraminidases by oseltamivir: A comparative structural analysis by the ScrewFit algorithm, Biophys. Chem 141: 117-123, 2009*).*

The results obtained with the shikimic acid were less promising, since there was no viral replication inhibition up to concentrations of 300 *µ*g/ml (Figure 2). This also shows that no a correlation between the amount of virus and doses of compound tested.

These results demonstrate that even though the antiviral can be effective for certain viruses, it does not mean that is true for other related viruses, not even under appropriate *in vitro* conditions.

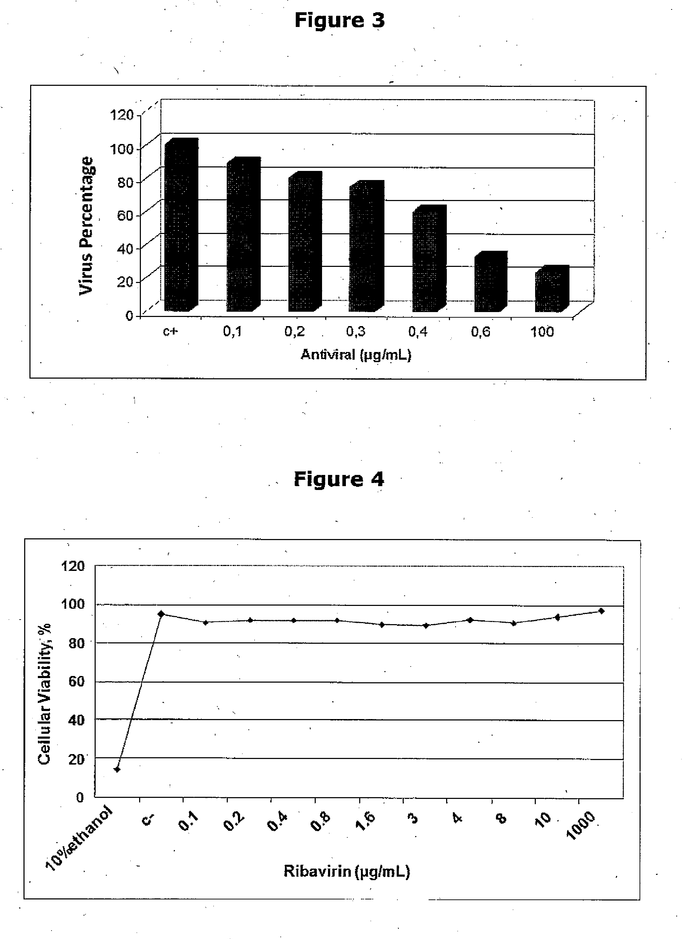

Otherwise, by subjecting the cells infected with ISA virus at different concentrations of the antiviral (ribavirin), an inhibition of virus replication was observed, which increased as the compound concentration was increased (Figure 3). The concentration which inhibits 50% of ISA virus replication (EC₅₀) was about 0.4 *µ*g/ml. This means that at fairly low concentrations of Antiviral an effective inhibition of virus replication is obtained.

As already discussed, a positive result in the *in vitro* inhibition for ISAV does not suggest that the compound is effective for the treatment of disease caused by the same virus in fish.

### DETERMINATION OF TOXICITY

The cytotoxicity of the compounds was assessed by cell viability assays. All assays were performed in SHK-1 cell cultures in duplicate.

In order to evaluate the cytotoxic effect of antiviral compounds on cell viability, monolayers of SHK-1 cells were grown in culture individual wells of 9.6 cm², up to a confluence of about 90-100%. In order to obtain a constant number of cells or a culture in growth steady state, fetal bovine serum (FBS) was removed from the culture medium. To each well a solution of the antiviral compound at different concentrations was added. Cells were incubated for 3 days at 15 °C. The state of the cell monolayer was observed in the inverted light microscope. The cells were subsequently washed twice with PBS, and then sprung with 200 *µ*L of 0.5 mM EDTA and 0.02% trypsin and incubated for 2 minutes at room temperature. The cells were centrifuged for 10 minutes at 3000 rpm, the supernatant removed and re-suspended in PBS 2% FBS. 3 *µ*L propidium iodide (50 *µ*g/mL in phosphate buffer) were added to 200 *µ*L of cell suspension and a cytometric counting of the viable and nonviable cells of a total of 100,000 cells was performed. The result was expressed as percentage of living cells (% cell viability).

Figure 4 shows the effect of increasing concentrations of Antiviral (ribavirin) on cell viability. The cells remained intact, close to 100% up to a concentration of at least 1 mg/mL Antiviral.

In these experiments, it was found that the concentration that produces 50% of cell toxicity (CC₅₀) was upper than 400 mg/mL, a valor much higher than the EC₅₀ values found (Figure 3).

With these results is not yet possible to ensure that the antiviral is effective in the treatment of disease caused by the ISA virus in salmonids, because while it is very promising that the compound efficiently inhibits viral replication *in vitro* and does not have cytotoxicity in concentrations up to 10⁷ times higher than the actual, it is not possible to extrapolate its utility *in vivo.*

Since ribavirin is approved for use in humans by international regulatory agencies,. such as Food and Drug Administration of the United Sates (FDA) and the European Medicines Agency (EMA), it is possible to guarantee some security for its use in fish.

### EXAMPLE 1:

### EVALUATION OF THE ANTIVIRAL EFFECT OF THE ORAL VETERINARY COMPOSITION COMPRISING RIBAVIRIN

Tests were conducted in a controlled seawater environment (25 ppt) with 156 units of smolt fish from the *Salmo salar* specie of about 70 g in weight, at the Universidad Católica de la Santísima Concepción de Chile.

The fish were distributed into its respective tanks and were left for 7 days to acclimatize before performing the process of infection. Fish were distributed in 5 independent systems with recirculating seawater, with two culture tanks, of 140 liters each one. Each line had a collector tank that distributed water to the closed system, and a biological filter to control nitrogen compounds.

The collector tank had a minimum capacity of 40 liters, from which the water is distributed to each of the tanks within the line. The tank should maintain conditions of pH, ammonium, and normal oxygenation accepted regularly by farms. The water distribution flow to the lines is consistent with a recirculation rate of 2 to 4 times / hour and a renewal rate of 10 to 30% of fresh water per day.

The temperature was kept between 14 to 16 °C. Abiotic factors such as pH and ammonia concentration were those normally accepted in Recirculating Systems of fish farms and were monitored with a biological filtration system.

The water circulation from the collector to the tanks was kept constant, maintaining a water recirculation rate of 2 to 4 times/hour, and was kept at the same rate between the tanks.

The fish were fed with 0.5% of their body weight of medicated feed or control feed.

After acclimatization they were separated into two groups of 30 fish each that remained uninfected. The first being the healthy control group (C1, see Table 2) and the second was the healthy control group treated with the highest antiviral dose (to assess toxicity) (C2, see Table 2).

The remaining 96 fish were divided into three groups of 32 fish each, and were placed in separate tanks-termed C3, T1 and T2. 40% of the fish from each of these tanks were injected intraperitoneally with a suspension of ISA virus and the remaining 60% were expected to get the infection by cohabitation. Thus the experimental infection resembles the reality at the farming.

The material that was in contact with the fish as thermometers, vacuum hoses, nets to move the fish, was properly separated and to each working group a set of exclusive use material was assigned.

Two of the three groups of infected fish were treated with the antiviral compound for 10 consecutive days (T1 and T2, see Table 2) and the other group was the untreated infected control group (C3). The treatment was started on day 11 post-infection and was provided with food. 2 doses of antiviral compound mixed with the feed were tested and also the excipient without the antiviral mixed with food.

Because 30 small fish were distributed per tank, with an average weight of 70 g, and that only 0.5% of body weight of medicated feed would be provided, the fish were not given normal food, in order to ensure that all supplied food would be ingest by them.

### REPARATION OF MEDICATED FEED:

Formulations of the antiviral were made available in order to prepare the medicated feed, as shown in the following Table 1:

**Table 1: Powder Formulations for oral administration of Ribavirin**

| **Description** | **%** | **Theoretical amount (g)** | **Weighed amount (g)** |
|---|---|---|---|
| Ribavirin base | 0.5 - 5 | 0.5 - 5 | 0.755 - 7.5 |
| *Starch 1500 | 99.5 - 95 | 99.5 - 95 | 149.5 - 142.5 |
| TOTAL | 100 | 100 | 150 |

| | | | |
|---|---|---|---|
| *Partially pregelatinized corn starch. | | | |

Four types of medicated feed were prepared, they are termed: Feed **F1,** Feed **F2,** Feed **F3** and Feed **F4.**

Feed **F1:** (400 *µ*g/kg) 0.084 g of 1% Antiviral formulation was mixed with 10.21 g of food. The mixture was vigorously stirred in an inflated polyethylene bag and then 0,210 mL of vegetable oil was added. It was stirred again until complete homogenization.

Feed **F2:** (800 *µ*g/kg) The same procedure as **F1** was used but adding 0.168 g of 1% Antiviral formulation.

Feed **F3:** (1,600 µg/kg) The same procedure as **F1** was used but adding 0.336 g of 1% Antiviral formulation.

Feed **F4:** 0.336 g of the excipients used in the formulations of the antiviral powder were weighted and mixed with 10.21 g of food. After vigorously stirring in an inflated polyethylene bag, 0.210 ml of vegetable oil were added. It was stirred again until complete homogenization.

The experimental design is summarized in the following Table 2:

**Table 2: Distribution of different assays and its encodings are listed.**

| **Tanks** | **Treatment** | **Antiviral dose (µg/kg)** | **Medicated feed** | **Duration of treatment (days)** | **N° fish** |
|---|---|---|---|---|---|
| C1 | Healthy control | 0 (only excipients) | F4 | 10 | 30 |
| C2 | Healthy control + oral treatment | 1600 | F3 | 10 | 30 |
| C3 | Infected control | 0 (only excipients | F4 | 10 | 32 |
| T1 | Infected + oral treatment | 800 | F2 | 10 | 32 |
| T2 | Infected + oral treatment | 400 | F1 | 10 | 32 |

On day 11 post-infection, the first mortality in fish of the infected control tank (C3) was observed.

The "infected fish" died along from day 11 to day 17 post-intraperitoneal infection and affected to the fish of tanks C3, T1 and T2.

The "uninfected fish" died between day 22 and day 28-post infection and affected to the fish of tanks C2, C3 and T2.

This distribution of mortality turned out interesting because it was clearly separated into two stages. The fish that died first were the ones infected intraperitoneally and later the fish that were infected by cohabitation. This last one happened in the tanks C3 and T2.

After 10 days of oral treatment, the fish were kept in their designated tanks until the 30 day post-intraperitoneal infection, after which all surviving fish of the test were sacrificed.

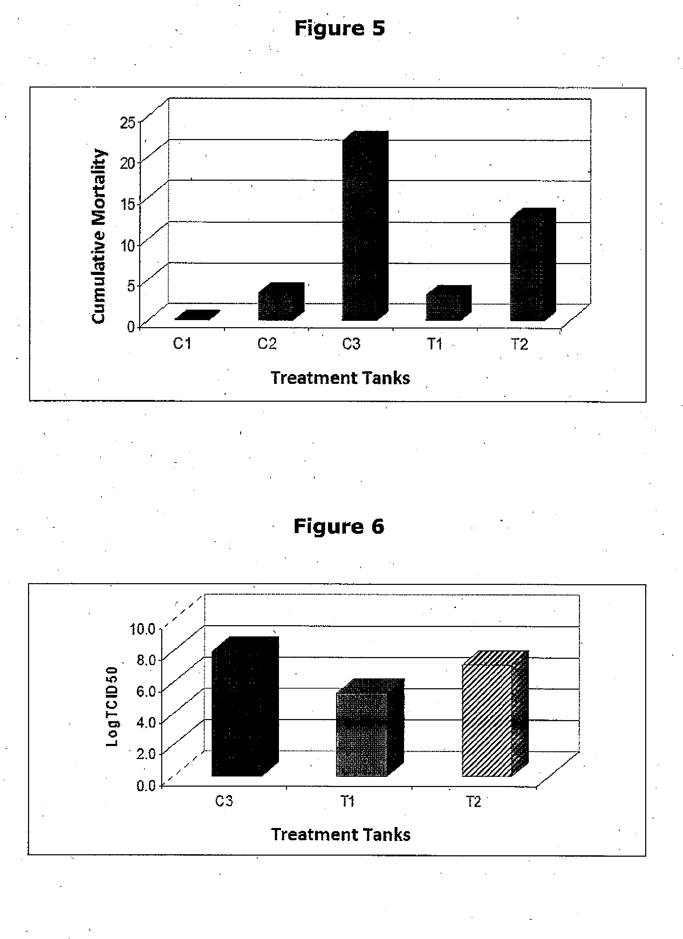

Cumulative mortality observed in the 30 days of the assay is plotted in Figure 5, which clearly shows that the C3-infected control with no treatment (positive control) was the one presenting the highest mortality rate reaching 21.9%.

In the control tank C1, which was not infected and not treated (negative control), no deaths were observed throughout the study period. Whereas in C2 control were not infected but received a dose of 1600 *µ*g/kg, a cumulative mortality of 3.33% was found.

In the tank where fish were infected and received a dose of 400 *µ*g/kg of antiviral (T2), fewer deaths than in the tank infected and untreated (C3) were observed, reaching 12.5% of mortality. The lowest mortality observed throughout the assay in infected fish was in the tank T2 which received 800 *µ*g/kg of antiviral, reaching a rate of only 3.1%.

These results show evidence that there was a clear development of the disease and that it'was effectively controlled with the administration of the antiviral.

Mortality levels expressed with different treatments showed a dose-response effect where the infected untreated tank showed the highest mortality (C3). The tank treated with the lowest dose of the antiviral (T2) had lower mortality than the untreated (C3), however it is still high and it is upper than the higher dose treatment (T1). The tank treated with the highest dose, however, had the lowest mortality (T2) and this is similar compared with the mortality of the controls.

With the control C1 the excipient safety is demonstrated. With control C2 the product safety is demonstrated when using a dose two to four times greater than those used in fish infected with ISAV.

These results show that it would be feasible to use even higher doses for the treatment of infected fish, although with the concentrations studied a really surprising effect is observed.

### ISA Diagnosis

Heart tissue was used as sample for the diagnosis of disease. It was stored in absolute ethanol or RNA later, if it were not immediately processed.

Molecular diagnosis was performed using RT-PCR in real time for ISA.

Presumptive diagnosis was performed by evaluation of clinical signs.

All analyzed fish samples from tanks C1 and C2 were negative for the presence of ISA virus. Thus, it was confirmed that the healthy controls not were clinically diseased by ISA, were negative for specific molecular diagnostic tests for the virus. It can be stated therefore, that there was no cross-contamination between infected and uninfected tanks.

### Determination of viral load

Segment 8 of the virus genome was used to quantify the number of copies of ISAV. The protocols are based on the description of Munir and Kibenge, Detection of infectious salmon anemia virus by real-time RT-PCR. J. Virol. Meth. 117, 37-47, 2004*.*

These results were expressed as TCID50 from the English 50% Tissue Culture Infective Dose, i.e. the amount of pathogen agent (in this case ISA virus) that is capable of producing pathological changes or cytopathic effect in 50% of the inoculated cells.

Figures 6-10 show the viral loads of fish subjected to treatments in tanks C3, T1 and T2. As shown in Figure 6, the average viral load in fish of the tanks T1 and T2 treated with the antiviral, was smaller than the uninfected fish and its magnitude was inversely proportional to the used dose.

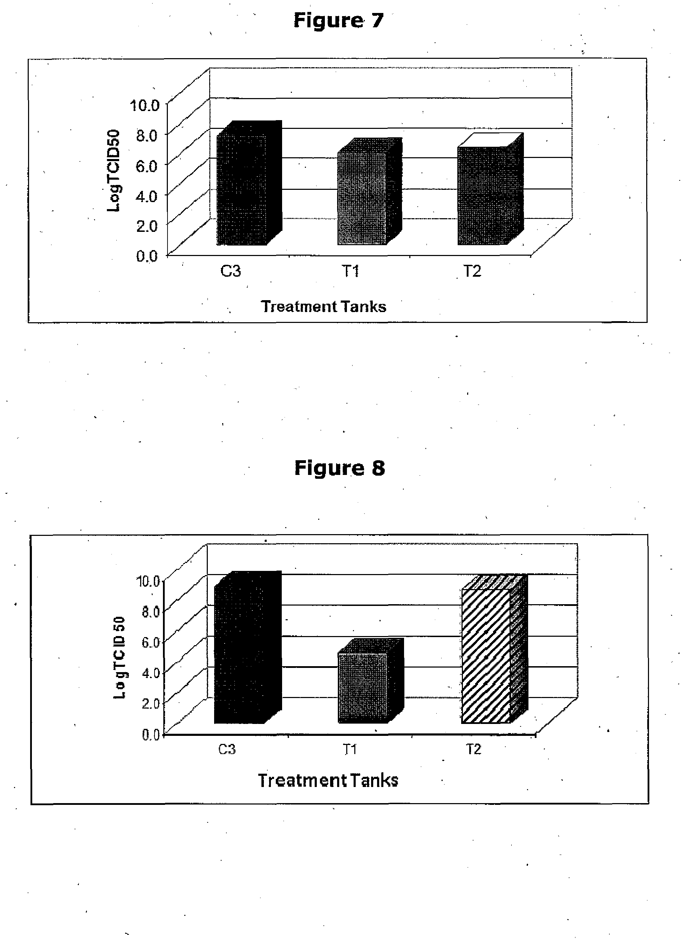

Viral load in live fish was very similar in the two treatment tanks T1 and T2 (Figure 7), and only slightly smaller than that of untreated infected fish (C3). In contrast, in the dead fish (Figure 8) viral load was significantly lower in fish treated with the highest dose of the antiviral (T1), compared with those that received the lowest dose (T2) and the ones with no treatment (C3). These results are consistent with the higher mortality rate observed in T2 and C3 groups.

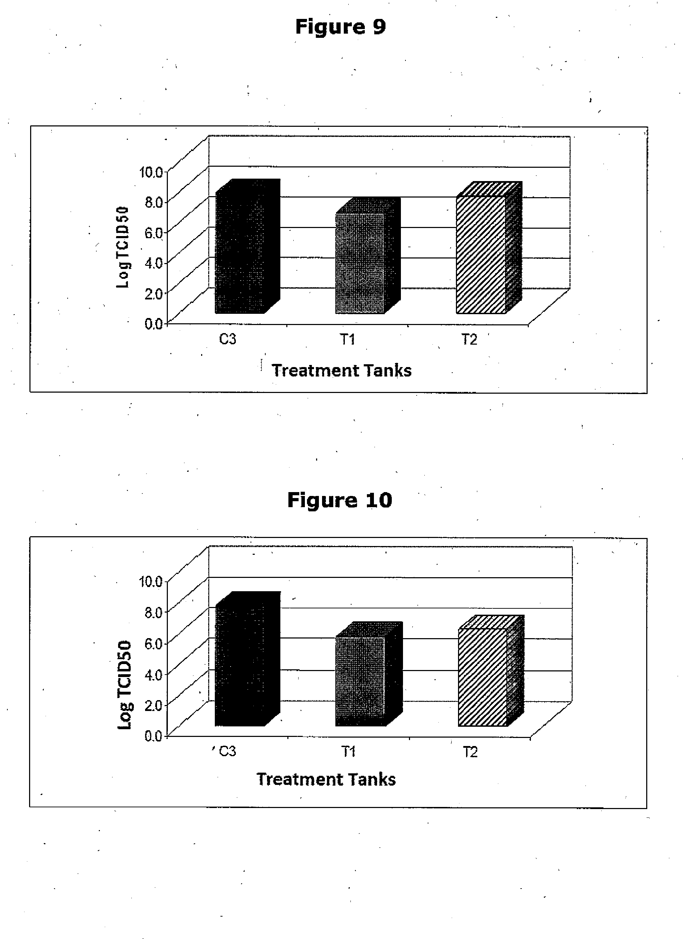

Similarly, in infected fish (Figure 9) the viral load was lower in the fish treated with the highest dose of the antiviral (T1), while the viral loads for fish treated with the lowest doses (T2) and the untreated (C3) were practically the same.

With the treatment of 800 *µ*g/kg of Antiviral no mortality was observed in fish that cohabitated, but the infection was confirmed because viral loads were detected, as shown in Figure 10 (T1). In the treatment with 400 *µ*g/kg a slightly higher viral load was observed in intraperitoneally uninfected fish (T2 in Figure 10) and it showed that they were finally infected by cohabitation with those infected.

Viral loads of treated fish (T1 and T2) were in all cases lower than that of the untreated fish (C3) and it was dose dependent.

### Necropsies Analysis

The necropsy of the fish in the assay of controlled environment was made through external and internal clinical analysis, with registration of morphometric parameters, description and registration of pathological features (necropsy itself), sampling of internal organs (heart, kidney, spleen and gills) in ethanol and RNA later, with counter samples (i.e. duplication of the samples of the organs same), along with the sample of muscle locket with skin to be used afterwards in analysis of deficiency (presence of the Antiviral in the muscle).

Heart samples of each fish were processed for the viral load analysis; the remaining samples were stored at -80 °C.

For purposes of nomenclature, 'live fish' is referred to the situation when the fish is taken alive but must be euthanized for delivery to the laboratory, and 'dead fish' is referred to mortality for ISA virus.

The fish were subjected to necropsy process mostly within 24 hours post sampling.

At the end of the assay all fish were euthanized and necropsied.

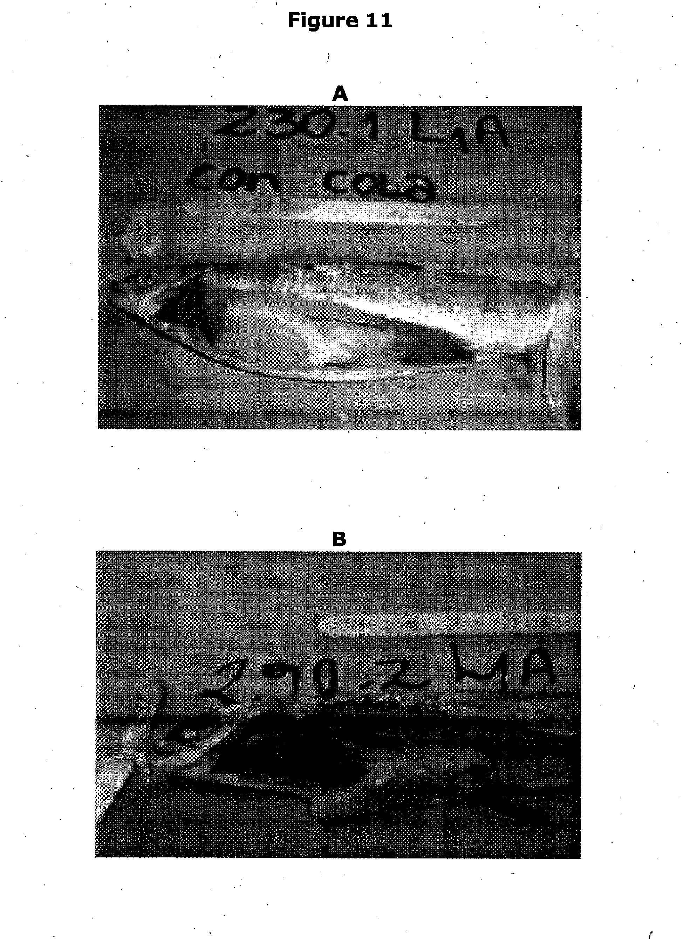

As shown in Figures 11A and 11 B, control fish of tanks C1 and C2 showed normal anatomic pathologic characteristics as well as gills with normal coloration. Internal organs such as liver, spleen and kidney were visualized as normal without inflammation. The coloration of visceral fat was whitish, showing normality.

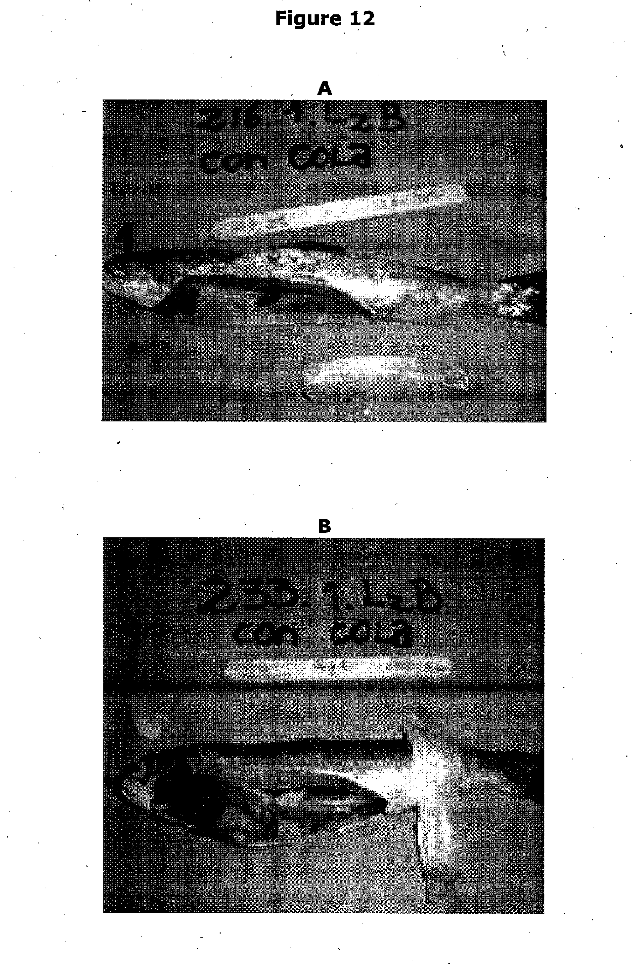

Similarly, fish of the control tank C2 showed normal anatomic pathologic features (Figures 12A and 12B) even Figure 12 B shows a fish with the stomach full of food, indicating a fish with appetite and high probability of good health.

All fish sampled from tanks C1 and C2 were negative for ISAV molecular analysis.

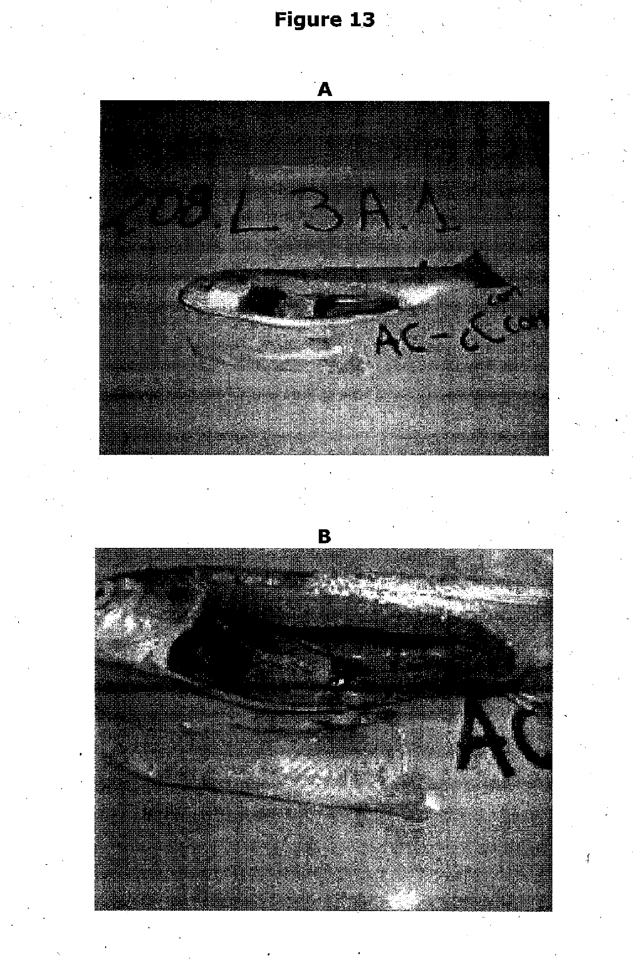

Figure 13 shows a fish infected with ISAV that did not received a treatment (tank C3). On day 11 of post intraperitoneal infection this first death occurred for this control. This fish showed characteristic clinical signs of ISA, that is dark burgundy liver with different shades of dark red, which corresponds to liver bleeding and hemorrhage in visceral fat usually as petechial (bleeding in point), within the most distinctive. Figure 13B is the close-up picture of Figure 13A.

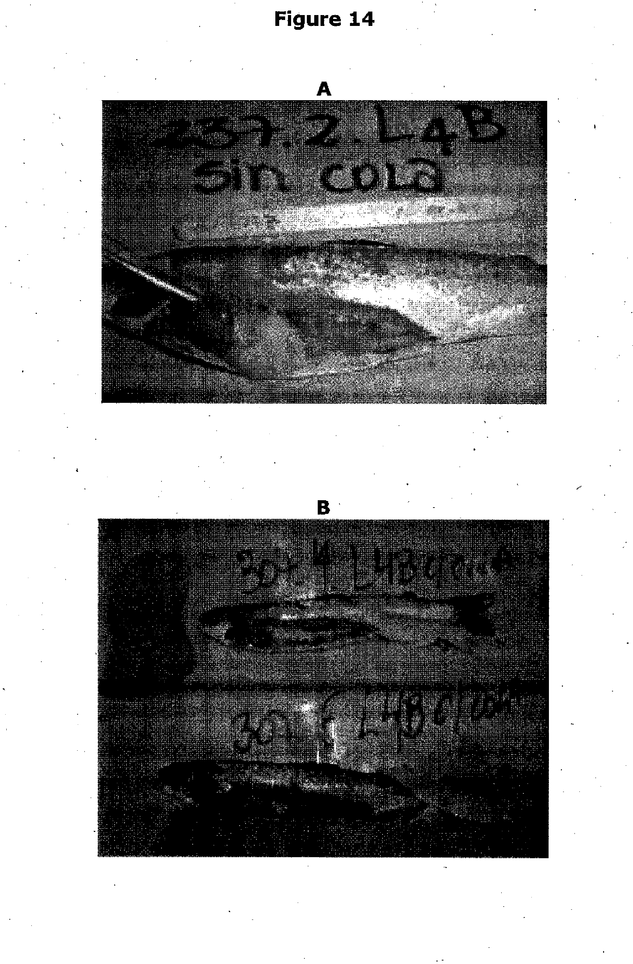

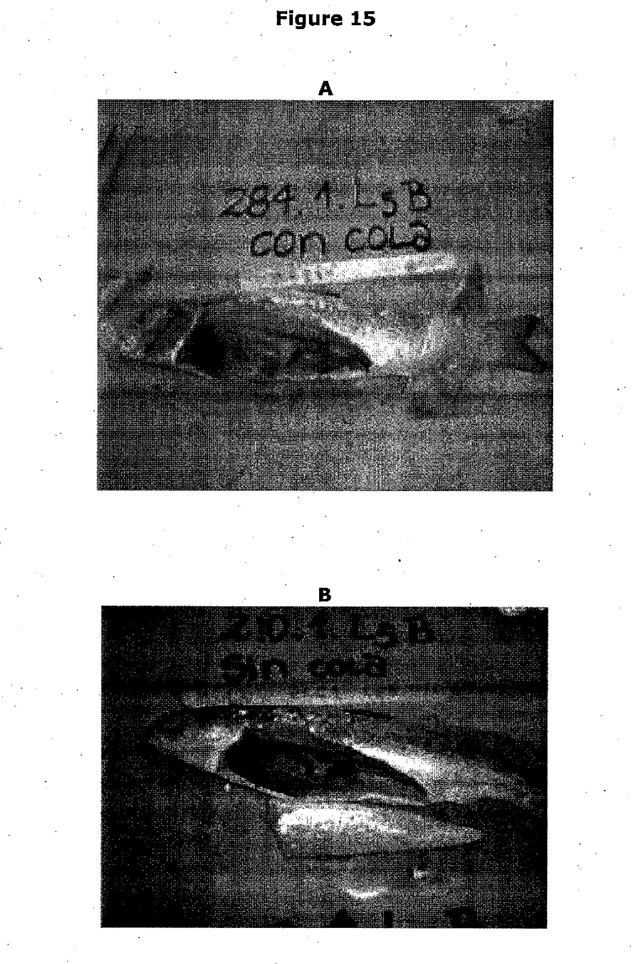

Figures 14 and 15 show infected fish subjected to treatment with the antiviral from tanks T1 and T2. In general they presented mild inflammatory signs.

Figure 15 A shows a fish from tank T2 that developed ISA and it was subjected to treatment with antiviral. It is noted that it has a stomach with abundant food, the liver looked red but its aspect was closer to normal. It is likely that this fish had been in a recovery process.

It was observed that at necropsy samples of "live" fish, the anatomic pathological findings were less severe, contrary to what was found in autopsies of "dead" fish samples, where fish showed severe clinical signs of ISA.

All analyzed mortality showed clinical signs of ISA, which were more evident in fish that developed the disease for a longer period of time, i.e. those that were infected into the peritoneum respect to those infected by cohabitation. This might have been a result due to increased contact time with the virus.

In general the consumption of food was moderate, however, from day 21 post viral infection, an absence of food in the stomach of all fish sampled is evidenced (controls and treated). The previous day was the last day of treatment and therefore the administration of medicated feed with either excipient or with antiviral. This means that all fish were subjected to a change in their feeding, which requires a period of adaptation.

### Evaluation of the hematocrit

The fish hematocrit was determined for fish that remained alive in each treatment. It was found that the hematocrit always went down in moribund fish no matter what the death cause was.

As discussed, the anatomic pathologic findings were more severe at necropsy samples of fish "dead" than the ones "alive". It is therefore possible that the hematocrit of living samples could not be a definitive indicator for predicting ISA, but it actually is an important parameter for assessing the toxicity of the antiviral used.

The hematocrit normal value in salmon is fairly wide-ranging but a minimum of 44% and a maximum of 64% can be used as limits (according to information obtained from the Universidad Austral of Chile).

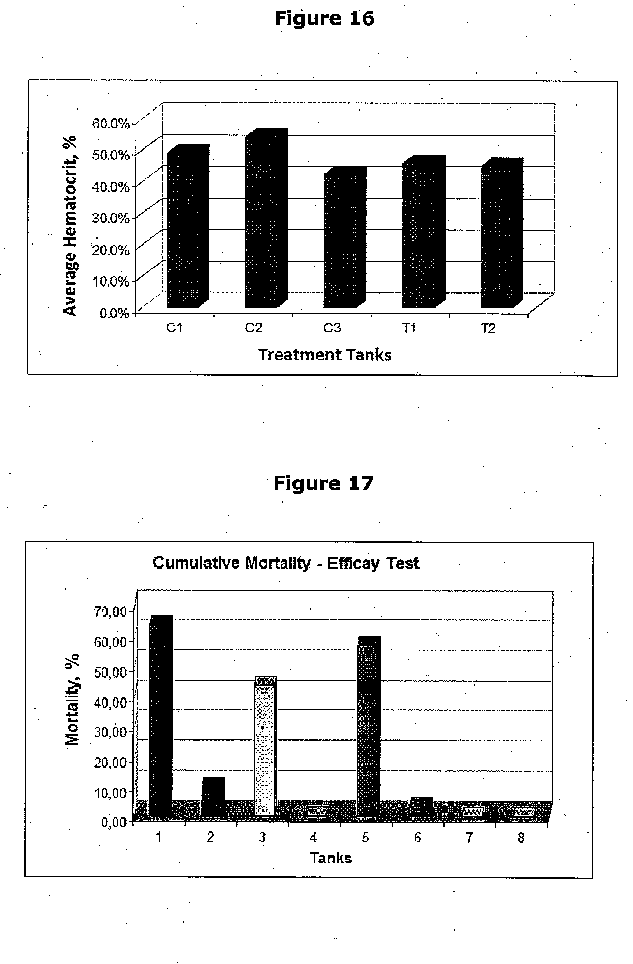

As seen in Figure 16, the average hematocrit for fish in tanks C1 and C2 was normal and similar from each other, with values close to 50%. These fish were the healthy controls, i.e. not infected with ISA virus.

The difference between C1 and C2 is given by the fact that the fish from tank C2 received a dose of 1600 *µ*g/mL of Antiviral, whereas the fish from the tank C1 did not receive the Antiviral but only the food with the excipients. It was noted that the control fish (C2) receiving the antiviral, showed a slightly upper hematocrit, 54%.

It is remarkable that the fish receiving a high dose of Antiviral, a compound characterized by its production of hemolytic anemia, showed no signs of developing anemia; on the contrary, their hematocrit remained at normal levels (C2).

The hematocrit value of blood samples of the fish from tank C3, which were infected with ISAV and did not receive the Antiviral, showed an average value of 42.1%. This leads to the idea that the hematocrit value may be defining in, live fish due to the anemia caused by viral infection.

Hematocrit values of fish from tanks T1 and T2, which were infected with ISAV and were given doses of 800 and 400 *µ*g/ml, respectively, showed values close to 45%, similar to each other and in turn within the normal range described. These values are, on average, higher than that observed in infected fish that did not receive the treatment (C3), which allows us to suggest that treatment with the antiviral would be producing a slight increase in the hematocrit value, a sign of improvement in the fish.

These results are truly amazing since, as mentioned earlier, it has been officially reported in international regulatory agencies that products containing ribavirin for human administration can produce hemolytic anemia; and on the other hand the disease caused by the ISA virus is characterized by showing severe anemia. That is by no means the effects found in the present invention could have been predicted.

The necropsies showed that the signs of the disease appear to be less severe in the treated fish and that the hematocrit results are slightly lower in untreated fish.

The results allow concluding that treatment with the antiviral compound significantly reduced mortality and even though it did not prevent the viral infection of cohabiting fish, it did decrease their viral loads and signs of the disease, allowing them to stay alive, active and being fed even in the presence of the virus.

### EXAMPLE 2:

### EVALUATION OF THE ANTIVIRAL EFFECT OF THE URAL VETERINARY COMPOSITION COMPRISING RIBAVIRIN

240 *Salmo salar smolted* fish with an average weight of 70 g were used. They were taken from the farm of the Eco Lago Verde belonging to the company Invertec.

Fish without record of disease by ISAV were selected, which was checked by sampling and subsequent analysis of molecular diagnosis by RT-PCR in real time. Additionally, fish were checked for bacterial and viral diseases. Before transferring the fish to the experimental station, 60 fish were sampled for a sanitary checkup, including necropsy analysis; fresh frotis of gills, intestine and skin; Gram staining of internal organs (spleen, kidney and brain), orange staining of acridine in gills, IFAT BKD for kidney, IFAT SRS for kidney, bacteriological cultures, RT-PCR for IPNv.

The fish were received in a "mother tank" of 4,000 L with saltwater at 25 ppt in recirculating flow of 120 L/min, at the experimental station of the Universidad Católica de la Santísima Concepción, in Concepción, Chile Several weeks after admission, fish were distributed in 8 tanks of 200 liters with 30 fish each, where they remained for the acclimatization period, inoculation, treatment and post-treatment monitoring. In this assay the acclimatization period was only 2 days, because the fish showed a very normal behavior and continued eating after being transferred from the mother tank. Since they had been for several weeks in the recirculation system of the Universidad Católica de la Santisima Concepción, fish were already acclimatized for this assay.

Fish were fed with 0.7 to 0.9% of body weight per day (bw/day) during the days of the assay. The days of treatment, the medication was incorporated in 0.5% bw adding 0.4 or 0.2% of normal food (without medication).

The diet used had the following proximal formula: 50% proteins, 21 % fat, 11% carbohydrates, 8% moisture, 0.7% fiber, 10% ash and 22 MJ per kg of gross energy.

In this period the fish were observed daily and any abnormal behavior was recorded. A daily record of the appetite of the fish (good, fair or poor) was also realized. Feeding was made by hand, in a controlled AM ration.

A Chilean viral isolate was used for experimental infection with ISA virus, which was sequenced in the hyper variable region (HPR) resulting in HPR of the type 7b, as the vast majority of isolates in our country. This was performed by intraperitoneal injection into the ventral line at a rate of 0.2 mL of inoculum per fish which had a titer of 104 TCID50 according the description by Jorgensen et al, Gene Expression Analyses in Atlantic salmon Challenged with infectious salmon anemia virus reveal Differences Between Individuals with early, intermediate and late Mortality, BMC Genomics 9, 179-194, 2008 and by Mjaaland et al, Susceptibility and immune responses Following experimental infection of Atlantic salmon MHC support (Salmo salar L.) with different infectious salmon anemia virus isolates, Arch Virol 150, 2195-2216 2005*.* The 40% of the fish in each tank was infected; assuming infection by cohabitation for the remaining 60%, by this means the infection is similar to what happens in the field. Additionally the control groups were inoculated with culture medium without the virus, so that all the fish were subjected to the same management. To perform the described procedure, fish were anesthetized with benzocaine in a tub separately with a dose of 40 to 60 ppm, and then the fish were recovered in fresh water without benzocaine and transferred to their original tank, where they stayed during the assay.

The administration of the veterinary composition comprising 5% of the antiviral ribavirin was given orally for 10 days. Assuming that viral replication will arise between 2 and 8 days post infection as described by *Totland et al.* (1996) thus avoiding the spread inside the fish and among fish. Three series of the medicated feed were prepared containing 1600 µg/kg of the antiviral compound.

During the time of the assay the physicochemical parameters were measured, such as ammonium, nitrite, nitrate, OD, T °, Salinity, at a daily basis. Regarding the mortality, it was verified that the cause of mortality was by infection with ISA virus and was discarded the action of any other pathogen.

From the generated mortality, the fish were sent to the laboratory for performing the necropsy analysis, viral loads, isolation and identification of the pathogen.

Once the treatment is finished, the fish remained for a period of 12 days in the tanks in order to follow the course of the post-treatment infection.

The fish were distributed as follows:
**Tank 1**: 30 infected fish without treatment, with food plus excipient.
**Tank 2:** 30 infected fish treated with medicated feed series number 1.
**Tank 3:** 30 infected fish without treatment, with food plus excipient.
**Tank 4:** 30 infected fish treated with medicated feed series number 2.
**Tank 5:** 30 infected fish without treatment, with food plus excipient.
**Tank 6:** 30 infected fish treated with medicated feed series number 3.
**Tank 7:** 30 uninfected fish and without treatment.
**Tank 8:** 30 uninfected fish and without treatment.

To assess the efficiency of the treatment with the veterinary composition comprising 5% of ribavirin, the assay was performed as follows:
a) Daily records of mortality
b) Quantification of viral load in dead fish
c) Necropsy of dead fish
d) Record of daily observation of the general behavior of the fish in terms of:
   a. Appetite, categorized as: good, fair, poor
   b. Activity, categorized as: active, torpid, bordered
e) Record of physicochemical parameters
f) Random sampling of fish from each tank in order to extract them blood for analysis of hematocrit and hemoglobin.
g) Viral isolation of some samples of dead fish

To evaluate the efficiency of the treatment, the following criteria were used:
a) The nonspecific mortality rate should be less than 5%. Above this percentage the test would be invalidated.
b) The record of mortality in infected fish with ISA virus, but not treated, must be equal or greater than 40% of the fish.

If mortality occurs it is recorded and removed to perform analysis of:
a) Necropsy for each dead fish
b) RT-PCR of ISA virus

### Previous analysis performed at baseline

The detailed results of the analysis of fresh smears of gills, skin and intestine, Gram staining and acridine orange, bacteriological tests including IFAT for BKD and viral analysis by RT-PCR of IPNV and ISAV performed before the assay are summarized below:

The **fresh smear of gills** evidenced mainly: absence of parasites and fungi in all samples analyzed; moderate hyperplasia (gill tissue inflammation) (grade II to III, mostly) and abundance of organic matter, which may be because fish had recently consumed food and due to the environmental conditions in the tanks.

The **fresh smear of skin and intestine** revealed no parasites in all samples analyzed.

**Gram staining** of smears of spleen, kidney and brain of all sampled fish showed no bacterial structures.

**Acridine orange staining** of smears of gills of all fish sampled showed no bacterial structures.

**IFAT/ BKD analysis** showed no Renibacterium salmoninarum in all samples analyzed.

**The bacteriological analysis** showed no bacterial growth from the analyzed samples of kidney, brain (TSA), spleen and gills (TYES).

The **RT-PCR** for **IPNV** analysis revealed that only one of the samples tested was slightly positive (1 pool of 12 pools in total).

The **RT-PCR analysis** for **ISAV** showed no ISA virus in all analyzed samples.

The SRS analysis was not performed because the fish came from fresh water.

In conclusion, despite some anatomic pathological characteristics altered that are described in the autopsies, the general appearance of the lot is relatively good and is within the use in the industry, so fish are considered representative of the Chilean industry. This also includes the presence of some fish carrying IPNV in the selected group.

Therefore it was considered that the fish were eligible for the assay.

### Daily mortality

Tanks No. 1, No. 3 and No. 5, correspond to assays performed with fish inoculated with ISA virus, with administration of food with excipient, i.e. they are the tanks with untreated fish. For them mortality began on day 11, day 4 and day 25 post inoculation, respectively.

The highest number of deaths at these tanks was found in the period defined as "treatment."

Tanks No. 2, No. 4 and No. 6 correspond to the tests with fish treated with the oral veterinary composition comprising ribavirin of the production series 1, 2 and 3, respectively. In these tanks there was not a development of the disease in the treatment period as it was observed in the tanks without treatment.

The distribution of mortality over time is shown in Table 3a and Table 3b. The difference in mortality rates in untreated fish over the ones with treatment can be clearly seen in Figure 17.

All dead fish during the assay were subjected to necropsy test and viral load analysis.

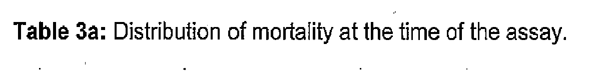

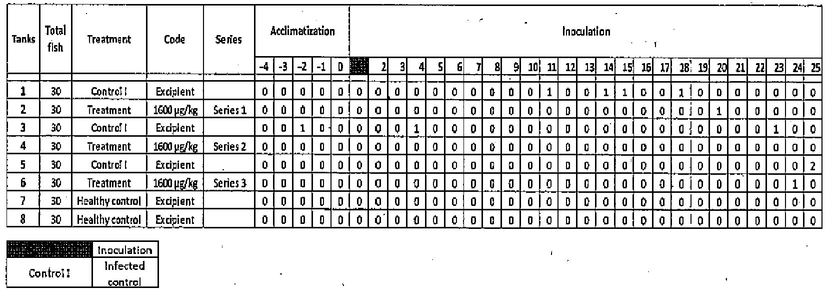

**Table 3b: Distribution of the mortality at the time of the assay**

| **Total fish** | **Treatmeat** | **Code** | **Series** | **Treatmeat** | | | | | | | | | | **Post-Treatment** | | | | | | | | | | | | **Total dead** | **Cumulative Mortality%** |
|---|---|---|---|---|---|---|---|---|---|---|---|---|---|---|---|---|---|---|---|---|---|---|---|---|---|---|---|
| | | | | **1** | **2** | **3** | **4** | **5** | **6** | **7** | **8** | **9** | **10** | **1** | **2** | **3** | **4** | **5** | **6** | **7** | **8** | **9** | **10** | **11** | **12** | | |
| | | | | | | | | | | | | | | | | | | | | | | | | | | | |
| 30 | Control I | Excipient | | 1 | 2 | 0 | 2 | 0 | 0 | 0 | 0 | 1 | 1 | 1 | 0 | 1 | 1 | 0 | 1 | 0 | 0 | 0 | 0 | 4 | 0 | 19 | 63.33 |
| 30 | Treatment | 1600µg/kg | Series 1 | 0 | 0 | 1 | 0 | 0 | 0 | 0 | 0 | 0 | 0 | 0 | 0 | 0 | 0 | 1 | 0 | 0 | 0 | 0 | 0 | 0 | 0 | 3 | 10.00 |
| 30 | Control I | Excipient | | 0 | 0 | 0 | 3 | 0 | 0 | 0 | 0 | 3 | 5 | 0 | 0 | 0 | 0 | 0 | 0 | 0 | 0 | 0 | 0 | 0 | 0 | 13 | 43.33 |
| 30 | Treatment | 1600µg/kg | Series 2 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0.30 |
| 30 | Control I | Excipient | | 0 | 0 | 0 | 6 | 0 | 1 | 0 | 0 | 0 | 2 | 1 | 0 | 1 | 0 | 1 | 0 | 0 | 0 | 0 | 3 | 0 | 0 | 17 | 56.67 |
| 30 | Treatment | 1600µg/kg | Series 3 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 1 | 3.33 |
| 30 | Healthy control | Excipient | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0.00 |
| 30 | Healthy control | Excipient | | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0 | 0.00 |

### Necropsies

It was possible to observe the appearance of ill fishes with clinical signs of ISA with the following evidence: externally, the signs were nonspecific, such as lethargy, loss of appetite, dark coloration and death.

The occurrence of the first mortalities was followed by delivery to the laboratory for necropsy. Dead fish analyzed, expressed in general systemic involvement with various levels of hemorrhage at internal organs level, mainly at visceral fat level, digestive. system and particularly at the liver level; the most distinctive being the "red wine" colored liver in some fish, with different shades of dark red, even or mottled, which corresponds to hepatic hemorrhage; it was also possible to observe hemorrhage in visceral fat usually as petechiae (hemorrhage at one specific point), in addition to nonspecific signs such as absence of food in digestive tract hemorrhage and/or congestion of digestive tract and inflammation of internal organs, with varying intensity.

### Viral loads

Regarding viral loads, all the dead fish showed positive viral loads which confirms that the fish were developing the infection, although in some cases it was moderate. This was also confirmed by the isolation of virus in cell culture SHK-1 from organs, which was made for some fishes.

A remarkable result is that the samples from tanks belonging to healthy controls (live sample) were negative, and the viral load found in the only one dead fish from tank No. 6 is also remarkable. The result of the viral load of healthy controls showed that they did not have viral load, and therefore cross contamination between tanks is discarded. By comparing the positive results of the treated infected fish with the untreated infected fish, it is evident that viral load (copy number) tended to be lower in treated infected fish and also fewer fish die. Additional evidence is obtained by observing the results of the other live fish sampled from tank No. 4 (treated tank that had no mortality throughout the study period, but that was infected because it had viral load, although in low figures).

### Acclimatization

The fish had already made the process of adaptation to the tank system, in a mother tank, having expended therefore, an important period of acclimatization time in the site of the assay.

During the period we define as acclimatization, the fish were moved to the final assay tanks, and that is the reason why they had two days of fasting. There were 3 deaths by mismanagement, which were replaced immediately, so they are not counted as mortality.

### Inoculation

On day 10 post-inoculation the first bordered fish appeared in the infected tanks, being more repetitive in fish belonging to tanks No. 1 and No. 5.

The fish from treated tanks as well as the healthy controls showed good appetite and activity throughout the period, as shown in Tables 4a and 4b.

In Tables 4 to 6, the meaning of the abbreviations is as follows:

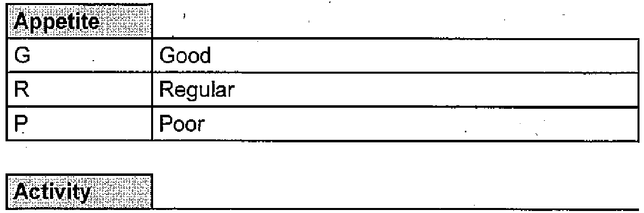

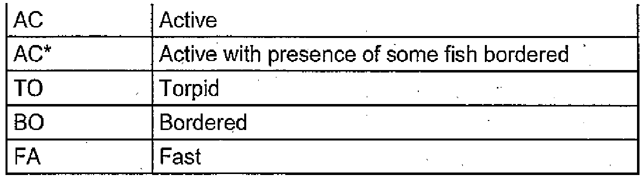

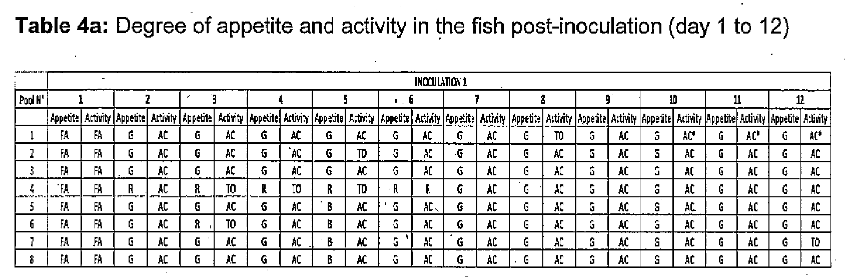

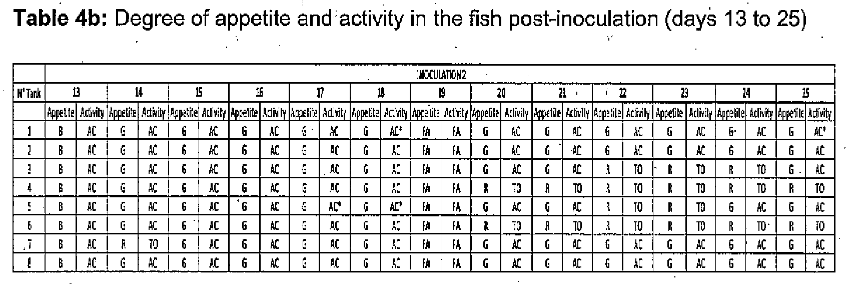

During the treatment time, all fish properly ingested the medicated feed, and the feed with excipient and no medication, However, some fish from tanks 3 and 5 showed slow food intake. Additionally the occurrence of bordered fish was persistent, as noted from the results shown in Table 5.

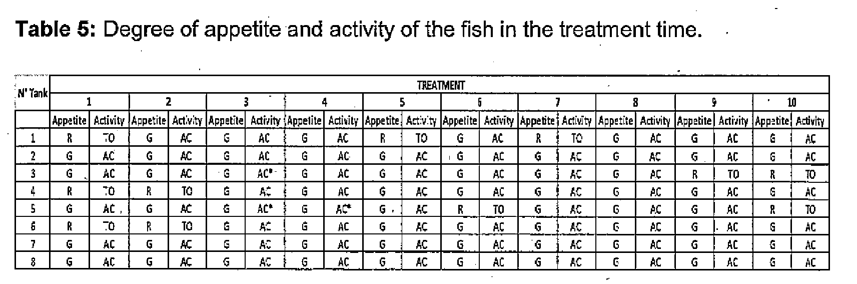

### Post-treatment

After treatment, the untreated infected fish (tanks 1, 3 and 5) persistently showed fish with appetite described as fair and poor as well as lethargy. On the other hand, fish belonging to the treated tanks showed instead better behavior and activity. These behaviors are demonstrated with the results shown in Table 6.

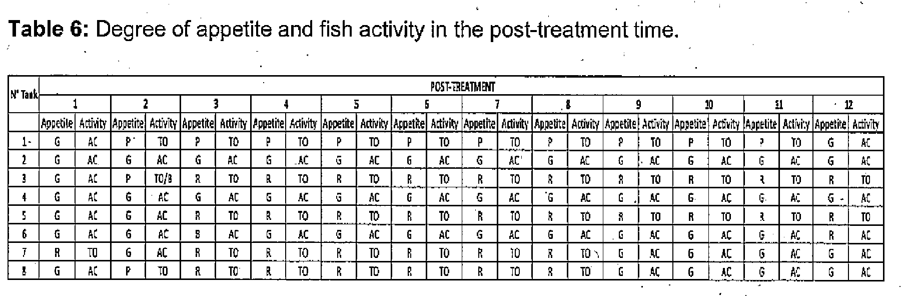

### Blood values

The hematocrit values obtained from blood of randomly sampled fish throughout the assay ranged moderately between 24 to 35.5%. However, the hematocrit average values of each treatment did not reveal the effect, since all values ranged around 30%.

The same tendency can be noted with the average values of hemoglobin (Hb).

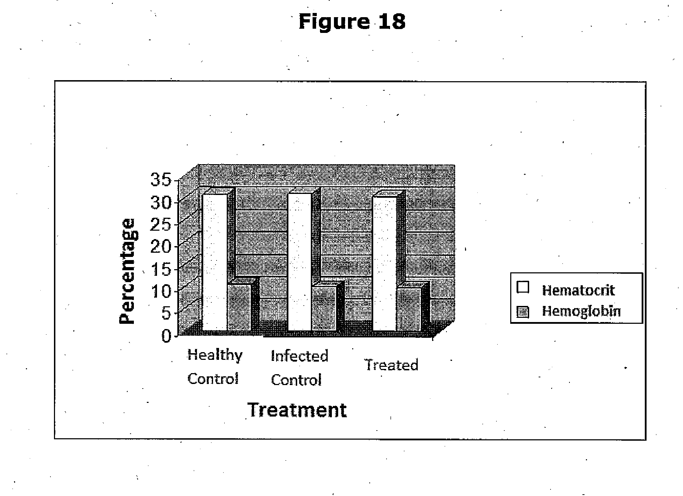

Thus it is concluded that the treatment has no side effects on blood hematocrit values and hemoglobin at concentrations levels tested (See Table 7 and Figure 18).

Individual tendencies for each treated tank showed that the treatment evidently did not produce a negative trend on blood values for hematocrit and hemoglobin.

**Table 7: Average results for values of hematocrit and hemoglobin in fish of the assay.**

| **Treatment** | **Hematocrit (%)** | **Hemoglobin (g%)** |
|---|---|---|
| Healthy control | 30.79 | 10.53 |
| Infected control | 30.92 | 10.09 |
| Treated | 30.31 | 10.03 |

### Physicochemical parameters

The water of the tanks was daily subjected to measurement of physicochemical parameters, such as nitrite, nitrate, ammonium, pH, temperature, and dissolved oxygen.

The physicochemical parameters were maintained within normal ranges, which are defined in Table 8.

**Table 8:**

| Nitrite (water) (mg/L) | Nitrite (seawater) (mg/L) | Nitrate (mg/L) | Ammonium (mg/L) | Ammoniac (mg/L) | pH | Temperature (°C) | Dissolved oxygen (mg/L) |
|---|---|---|---|---|---|---|---|
| <1 | <13 | 0 - 400 or more | <1 | 0.0125 | 6.5 - 8.5 | 10 - 18 | >5.5 |

In cases where the ammonium, ammoniac or nitrite were higher than the normal value specified in Table 8, a change of water was performed (mixture of water with salinity of 24 ppt).

The results shown above indicate that the criteria established for evaluating the effectiveness of treatment with the veterinary composition of 5% antiviral ribavirin were met, since the following results were obtained:
a) Zero percent of nonspecific mortality in uninfected and untreated tanks.
b) A range between 43% and 63% of mortality in untreated infected tanks and
c) A range between 0% and 3% of mortality in infected tanks treated with the antiviral composition.

In conclusion:
a) The non-specific mortality rate was less than 5% and specific mortality was greater than 40%, which validates the assay and,
b) The recorded survival for treated fish ranges from 100% to 84% compared to the one observed in untreated fish.

This study demonstrates the efficiency of the veterinary composition comprising 5% ribavirin and its effectiveness in the treatment of the disease ISAV affecting salmonids.

### INDICATIONS, DOSAGE AND ADMINISTRATION

From the results of the assay, it is possible to ensure that the oral veterinary composition comprising the antiviral has been specially formulated for the control of infectious salmon anemia, ISA, and that is very effective when administered at the early stage of the disease, in individuals with ISAV positive diagnosis without clinical symptoms or in individuals in the early stages of symptoms.

The recommended oral dose is 1.6 mg/kg body weight, during 10 days.

The veterinary composition in the powder form comprising the antiviral must be impregnated through an oily base to the feed. The dispense process should follow the pattern of feeding rate and should to be added to a suitable size of the food pellet according to the individuals to be treated.

The following Table 9 shows the dose related to the feed pellets size for oral administration of the composition of the invention:

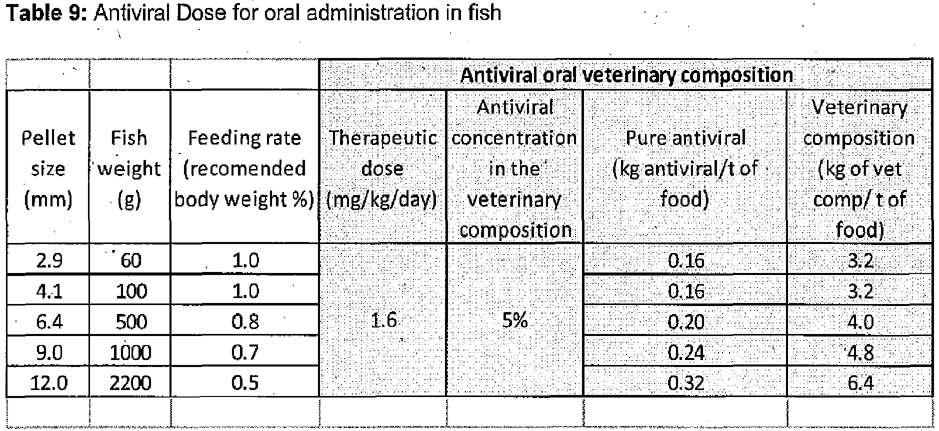

The veterinary composition of the invention is suggested to be supplied for 10 consecutive days, once a day, in the morning with a good demand for food, in order to facilitate its oral administration and the effectiveness of the treatment.

### Veterinary compositions according to the present invention

In the Table 10 is detailed the various developed formulations comprising the antiviral as active ingredient for a pharmaceutical form in powder, designed for use in an oily suspension or as a solid component in the formula of a medicated feed pellet. It also is showed the manufacturing process used for each composition..

**Table 10: Veterinary compositions comprising the antiviral and its manufacturing process.**

| **Nº Formula** | **Detailed composition and manufacturing process** |
|---|---|
| 1 | Powder mixture 1%, obtained by dry way. Geometrical dilution process of the active with Lactose Spray Dried. |
| 2 | Powder mixture 1%, obtained by dry way. Geometrical dilution process of the active with pre-gelatinized starch. |
| 3 | Powder mixture 1%, obtained by wet way. Granulation process of the active with alcohol in lactose monohydrate, combined later by geometric dilution of the granulated spray-dried lactose. |
| 4 | Powder mixture 1%, obtained by wet way. Granulation process of the active with alcohol in starch and then geometric dilution of the granulated in pre-gelatinized starch. |
| 5 | Powder mixture 1%, obtained by wet way. Deposition process by solvent in mixture of corn starch - pre-gelatinized starch. |
| 6 | Formula Nº 2, increasing size of the range to decreasing variability due to manufacturing process. |
| 7 | Powder mixture 1% obtained by dry way. Geometrical dilution process of the active with pre-gelatinized starch and colloidal silicon dioxide to improve uniformity content and flow. |
| 8 | Formula N°5, increasing size of the series. |
| 9 | Suspension W/O to 1% of the active in fish oil stabilized with emulsifier with low HLB value (Crill 4). |
| 10 | Powder mixture 0.5%, obtained by dry way. Geometrical dilution process of the active with pre-gelatinized starch. |
| 11 | Powder mixture 5%, obtained by dry way. Geometrical dilution process of the active with pre-gelatinized starch. |
| 12 | Powder mixture 10%, obtained by dry way. Geometrical dilution process of the active with pre-gelatinized starch. |
| 13 | Powder mixture 30%, obtained by dry way. Geometrical dilution process of the active with pre-gelatinized starch. |
| 14 | Impregnated pellet test. |

### Tests of pellet impregnation with the developed product (powder formulated)

With the objective of establishing a basic method of obtaining in a laboratory scale an impregnated pellet with the developed product, the formula 11 was used to prepare a suspension of the product in oil fish equivalent to 2% weight of the pellet to be impregnated.

For this purpose 500 g of pellet with a size 2.9 mm were used (Nutra Skretting Alpha Plus 60), which were impregnated in a coating process performed in traditional machine dragees under controlled operating conditions.

To this sample a method of preparation and extraction of the active was established, and it was analyzed using the defined chromatographic system.

The implemented method proved to be selective for the analyte and for the degradation products obtained in the samples subjected to degradation protocol in different conditions.

As a result of the degradation tests for the analyte in solution, it was determined that the active substance is sensitive to degradation in alkaline medium at room temperature and catalyzed by temperature, the decrease being approximately 36 and 88%, respectively. In acidic medium, degradation catalyzed by temperature was observed, reaching a decrease of 38% of the initial concentration. No degradation products were observed due to photolysis or oxidation.

Likewise, the study establishes stability of the aqueous solution of the analyte, if it is kept refrigerated until 48 hours post processing.

Table 11 shows the results obtained from samples of raw material and product when autoclaved at 121 °C with 15 psi of pressure (1 atmosphere) and 25% relative humidity.

**Table 11: Results from the degradation of raw material (antiviral ribavirin), and the formulated product with starch, when autoclaved at 121 °C with 15 psi of pressure (1 atmosphere) and 25% relative humidity.**

| **Raw material sample (antiviral)** | **% Antiviral** | **RSD** |
|---|---|---|
| RM 5 minutes in autoclave | 97.2 | 0.8 |
| RM 10 minutes in autoclave | 91.9 | 5.5 |
| RM 30 minutes in autoclave | 90.3 | 11.7 |
| RM 20 minutes drying + 30 minutes in autoclave | 99.5 | 9.4 |
| | | |

| **Raw material sample (antiviral) + starch** | | |
|---|---|---|
| 10% antiviral 5 minutes in autoclave | 85.0 | 6.1 |
| 10% antiviral 10 minutes in autoclave | 99.0 | 30.4 |
| 10% antiviral 30 minutes in autoclave | 109.0 | 10.1 |
| 10% antiviral 20 minutes drying + 30 minutes in autoclave | 102.0 | 24.9 |

The results suggest that the active principle is resistant to a production process, which includes a short time exposure to high temperature and relative humidity.

### Results of various veterinary compositions tested:

Table 12 shows the titration results obtained from the various compositions tested with the analytical method implemented. These compositions correspond to those detailed in Table 10.

**Table 12: Average titration and relative standard deviation (RSD) of various compositions developed in the present invention.**

| **Formula Nº** | **Average titration (%)** | **RSD (%)** |
|---|---|---|
| 1 | 76.5 | 1.45 |
| 2 | 95.5 | 0.91 |
| 3 | 87.2 | 0.37 |
| 4 | 86.2 | 2.8 |
| 5 | 98.9 | 1.1 |
| 6 | 96.6 | 0.73 |
| 7 | 88.7 | 1.19 |
| 8 | 101.7 | 2.06 |
| 11 | 100 | 0 |

The results obtained in the analyzed series showed a better behavior of the product in solid pharmaceutical form (formulas 6, 8 and 11), which were made with pre-gelatinized starch as the base of the excipient obtained by dry way and geometric dilution of the active in the excipient. The differences obtained with respect to the theoretical value might be explained by losses during the process or lack of uniformity (insufficient mixing).

From the galenic point of view, formula 11 provides a better suspensibility in oil phase (fish oil), and also showed good analytical results by titration. The obtained suspension is capable of maintaining without sedimentation for at least 5 minutes with no stirring. If this process is maintained during application of the product on the pellet no sedimentation occurred.

As a result of the impregnation test, it becomes apparent the convenience of having a process of preparation of suspension with the oily phase kept at room temperature and permanent stirring until the time of application in the pellet. The used mixing process (machine dragees) proved to be efficient in the process model implemented in a laboratory scale.

## Claims

1. Oral veterinary composition ,for salmonids comprising 1-beta-D-ribofuranosyl-1*H-*1,2,4-triazole-3-carboxamide and acceptable veterinarily excipients.

2. Oral veterinary composition for salmonids according to claim 1 wherein 1-beta-D-ribofuranosyl-1*H*-1,2,4-triazole-3-carboxamide is present in amounts ranging 0.5% to 5% by weight relative to the total weight of the composition.

3. Oral veterinary composition for salmonids according to claim 1 wherein acceptable veterinarily excipients are present in amounts ranging 99.5% to 95% by weight, relative to the total weight of the composition.

4. Oral veterinary composition for salmonids according to claim 1 wherein acceptable veterinarily excipients are selected from: lactose spray dried, partially pregelatinized corn starch, lactose monohydrate, corn starch, colloidal silicon dioxide, or mixtures thereof.

5. Oral veterinary composition for salmonids according to claim 1 wherein an acceptable veterinarily excipient is partially pregelatinized corn starch.

6. Oral veterinary composition for salmonids according to claim 1 wherein acceptable veterinarily excipient is partially pregelatinized corn starch in amounts ranging 99.5% to 95% by weight relative to the total weight of the composition.

7. Oral veterinary composition for salmonids according to claim 1 wherein the composition is mixed with fish feed pellets.

8. Use of the oral veterinary composition for salmonids according to claim 1 in the manufacture of a medicament for the treatment of infectious salmon anemia (ISA) in salmonids.

9. Use of the oral veterinary composition for salmonids according to claim 8 wherein the medicament is mixed with fish feed pellets.
